Europäisches Patentamt

European Patent Office

Office européen des brevets

(19)

(11) Publication number: **0 023 731**
A2

(12) # EUROPEAN PATENT APPLICATION

(21) Application number: **80200653.6**

(22) Date of filing: **04.07.80**

(51) Int. Cl.³: **A 61 K 39/295, C 12 N 7/00**

(30) Priority: **17.07.79 GB 7924916**

(43) Date of publication of application: **11.02.81**
**Bulletin 81/6**

(84) Designated Contracting States: **AT BE CH DE FR GB IT**
**LI LU NL SE**

(71) Applicant: **Gist - Brocades N.V., Wateringseweg 1 P.O.**
**Box 1, NL-2600 MA Delft (NL)**

(72) Inventor: **Bijlenga, Gosse, 4, Allée du Bel Horizon,**
**F-69890 La Tour de Salvagny (FR)**

(74) Representative: **Van der Straaten, Jan Anthony et al, c/o**
**GIST-BROCADES N.V. Patents and Trademarks**
**Department Wateringseweg 1 P.O. Box 1, NL-2600 MA**
**Delft (NL)**

(54) **Process for the preparation of polyvalent virus vaccines in a single cell system, such polyvalent vaccines and their application.**

(57)    A polyvalent virus vaccine comprising a rabies virus and a canine distemper virus, and substantially free from tissue material, is prepared by propagating the viruses in a single cell system.

Preferably the rabies strain no. 675, deposited with the Czechoslovak National Collection of Type Cultures of the Institute of Hygiene and Epidemiology in Prague under CNCTC no. A 04/77, is used for this purpose.

The preparation process is preferably carried out by first infecting a suitable single cell system with a rabies virus strain, propagating the rabies virus and then infecting the cell system with at least a canine distemper virus, propagating the viruses and preparing from the propagation product the polyvalent vaccine.

The so abtained polyvalent virus vaccine may be used for a method for immunisation of warm blooded animals comprising inocculation of the animal with such vaccine.

- 1 -

Process for the preparation of polyvalent virus vaccines in a single cell system, such polyvalent vaccines and their application.

This invention relates to a novel process for the preparation of polyvalent virus vaccines in a single cell system, to polyvalent virus vaccines produced according to this process and to the application of these virus vaccines for immunization of warm blooded animals.

More particularly, this invention relates to a novel process for the preparation of polyvalent virus vaccines, which comprises at least a living rabies virus strain and a living canine distemper virus, by propagation of the virus strains in a single cell system.

The possibility of the production of the polyvalent vaccine according to the invention is based on the principle of infection of the same cells with two or more different types of viruses.

There are considerable advantages in the production of polyvalent virus vaccines by culture of more than one type of virus in the same cell system, for example:

1. Lower production expenses due to lower labour costs and the lower cost of culture media, glasswork, lyophilization, diluents, and packing material, resulting in a decreased market price of the polyvalent vaccine, and

2. Application of the vaccine to dogs, minks, ferrets and the like by a single inoculation instead of two or more.

In addition, the use of a single cell system for culture of the viruses avoids the general disadvantages associated with the use of, e.g. chick embryos, i.e. the presence of extraneous animal material in the final product, which causes known undesired side-effects.

From Can.J.Comp.Med.Vet.Sci., 28, February 1965, p. 38-41, it is known to grow simultaneously, rabies virus and canine distemper virus, in the same chick embryos. It is, however, clearly indicated that in fact both viruses are inoculated on different tissues of the embryonated eggs, and thus do not multiply in the same cells.

The viruses used were Flury rabies virus (RV) of chick embryo passage between 59 and 61, and canine distemper virus (CD) of chick embryo passage between 55 and 59, while Salmonella-pullorum-free 5 to 6 days old embryonated eggs were selected.

Although it is indicated that bivalent vaccines for rabies and canine distemper could be produced, of which the potencies and virus titres were comparable to those of rabies vaccine and canine distemper vaccine, produced

- 3 -

separately, these obtained bivalent vaccines have the general disadvantages as known in the art of vaccine preparation, due to the extraneous tissue material, which causes the generally known undesired side effects.

From British Patent No. 1270918 it is known to prepare multivalent vaccines in a single cell culture by simultaneous cultivation of different viruses, causing respiratory diseases, especially such viruses as are selected from the group consisting of a) respiratory syncytial virus b) parainfluenza viruses c) influenza viruses strains A and B d) infectious bronchitis-"like" virus and e) mycoplasma pneumoniae.

However in the description of the invention on page 1, lines 80-87, it is clearly stated that it was originally believed by people skilled in the art, that infection of a cell with one virus type might preclude simultaneous infection of the same cell with a different virus although later on this theory was tested and proved not to be generally true.

Nevertheless multiple infection of cells has only been shown to be feasible with certain viruses, and is not generally applicable.

Therefore according to British Patent No. 1270918, specific combinations of viruses being rather similar in type seemed to be capable of being used for the simultaneous infection of cells and subsequent harvesting of multivalent vaccines. The application of the same process on viruses of really different characteristics, and which also differ from the types of viruses mentioned in British Patent No. 1270918, i.e. rabies and canine distemper, was certainly not described in, or suggested by, the disclosure of British Patent No. 1270918.

In particular, because of the difficulties associated with the simultaneous culture of two or more different viruses, all the presently marketed polyvalent vaccines are still prepared by mixing vaccines obtained individually by growing each virus vaccine in separate cell cultures.

Moreover, it will be appreciated that it is known from e.g. Avian Dis. 7, 106-122, 1963; Am. J. Vet. Res. 17, 294-298, 1956 and Avian Dis., 11 399-406, 1967 and Virology 33, 598-608, 1967 that mixtures of several live vaccines prepared separately and then mixed may lose the original activity of the separate components due to mutual inhibition. The problem of mutual inhibition may occur not only when separate vaccines are mixed, and the preparation of the combined vaccines of the invention during which no substantial interference of the viruses within the same cell system occurs, to yield a combined vaccine product having acceptable titres for both viruses, is surprising.

- 5 -

As a result of research and experimentation a process was surprisingly found for the preparation of polyvalent virus vaccines comprising at least a rabies virus and canine distemper virus, by propagation of these virus strains in a single cell system.

The present invention accordingly provides a process for the preparation of a polyvalent vaccine which comprises at least a living rabies virus and a living canine distemper virus by propagating the viruses in a single cell system and preparing from the propagation product the polyvalent vaccine. The process is preferably carried out by first infecting a suitable single cell system with a rabies virus strain, propagating the rabies virus, and then infecting the cell system with at least a canine distemper virus, propagating the viruses and preparing from the propagation product the polyvalent vaccine.

The invention further provides a polyvalent vaccine comprising a rabies virus and a canine distemper virus, and substantially free from material emanating from more than one type of tissue cell used to propagate the viruses. The vaccines preferably contain $10^{7.5}$ to $10^{9.2}$ p.f.u./ml of rabies virus and, preferably, $10^{4.5}$ to $10^{7}$ p.f.u./ml of canine distemper virus.

Any canine distemper virus and any rabies virus which can be propagated adquately _in vitro_ and which can be safely applied as a living vaccine, provoking an adequate protection, may be used in the combined rabies-canine distemper vaccine according to the present invention, such as the Wisconsin (FxNO) or Onderstepoort strains (canine distemper) and the Flury strains (rabies), and strains derived therefrom.

Preferably as rabies virus the strain no. 675 deposited with the Czechoslovak National Collection of Type Cultures of the Institute of Hygiene and Epidemiology in Prague under no. CNCTC A 04/77 and disclosed e.g. in the German patent application no. 2803240, is used. As canine distemper virus preferably the Onderstepoort avianized vaccine strain (J.Vet.Res. 1956, 27, no. 1,19-53) is used.

The time between infections with the two viruses depends on the multiplicity of infection (m.o.i.) of both types of viruses and the host cells employed. It was found that in vitro infection with the rabies virus is not followed by the production of interferon, which permits consecutive interferon-susceptible viral infections. Non-simultaneous infection appeared to be only possible in case the multiplication of the primary infection of the cells does not inhibit or interfere with the subsequent infection by another virus type.

Infection of a single cell system with two or more viruses of susceptible cells also depends on the

⌈Onderstepoort

problem of viral adherence to the total of cell surface available. In the case of rabies infection of BHK-21 cells, and a subsequent infection with the canine distemper virus a polycation is used to ensure adherence of the canine distemper virus to the BHK-21 cells.

It will be appreciated that eventually a third virus or additional viruses could be propagated in the single cell system used, such a procedure mainly depending on the absence of a) interfering substances and b) possible inhibition of production of specific viral products necessary for their multiplication.

For the propagation of a bivalent vaccine - rabies and canine distemper - in a single cell system, monolayer cultures may be employed. However, any other adequate system such as cell suspension cultures, cell-covered beads in suspension and multiplate cultures can be used for the propagation of the viruses in those cells. Such systems usually increase the virus yield due to the fact that more cells for infection and multiplication are available in a lower quantity of medium compared with stationary monolayer cultures.

According to a preferred process of the present invention, the polyvalent vaccines are for example prepared by growing the virus strains in monolayers of primary or secondary chicken embryo fibroblasts derived from SPF 10 days old chicken embryos or in monolayers

- 8 -

derived from other cells of avian origin or mammalian cells, e.g. the BHK-21 (Baby Hamster Kidney cell line - 21 passages). Also BHK-21 13S (13 passages in suspension culture) suspended cultures may be applied with good results.

In a preferred embodiment of the process of the present invention, culture cells, e.g. BHK-21 culture cells, are grown in monolayers for three to four days until confluency, preferably by means of BHK-21 medium containing 10% tryptose phosphate broth and 10% inactivated newborn calf serum in the presence of antibiotics (e.g. 100 IU of penicillin and 100 micrograms of streptomycin), the confluent monolayer is infected, preferably with the rabies vaccine strain 675, in a multiplicity of infection (m.o.i.) preferably between 0.01 and 1 per cell and incubated preferably for 45-60 minutes at 36°C. The virus-containing liquid is then removed, fresh maintenance medium [e.g. a BHK-21 medium and 0.2% bovine serum albumin fraction V with antibiotics (i.e. kanamycin 5% in an amount of 2ml/1000 ml of medium)] is added and the pH is adjusted to 7.5 to 8.3. After virus multiplication, preferably for 12-36 hours, the maintenance medium is removed and kept sterile for adding after infection of the cells with the canine distemper virus, e.g. the Onderstepoort avianized vaccine strain. The infection of the BHK-21 cells by the second virus is performed in the presence of a suitable polycation which is necessary for the adherence of the virus to them. As a suitable polycation, e.g. diethylaminoethyldextran is generally used in an amount of 50-200, and preferably about 100, micrograms/ml of medium. Without this polycation the canine distemper virus does not adhere to BHK-21 cells. The presence of the polycation may not be necessary for the adherence of the second virus to other cell types. The multiplicity of infection employed for the second virus is preferably 1-0.05, most preferably 0.5-0.05 per cell. After incubation, preferably for 1-2 hours at 36°C, the virus-containing fluid is removed and the original earlier collected maintenance medium is added to the cells.

One to four days later the cell sheet has been destroyed by the action of both viruses and the medium is harvested with the cells and frozen. Alternatively, after the distemper virus infection the cell sheets may be trypsinized and the infected cells put into spinner culture, preferably using 1-5 x 10$^6$ cells/ml of medium, preferably for 2 to 3 days at 33°C.

After thawing, which releases additional amounts of virus from the cells, the cell debris is removed by known methods, e.g. centrifugation or filtration through sterile filters of, e.g. 4 microns

- 10 -

pore size, whereafter the cell-free virus-containing liquid is kept at a temperature not exceeding -70°C. Other methods may be used to disrupt the cells after incubation to release additional amounts of virus, e.g. by subjecting the cell suspension to ultrasonic vibration. By the expression "known methods" as used in this specification is meant methods heretofore used or described in the literature.

It will be appreciated that the final polyvalent vaccines, prepared by the process of the present invention, may contain e.g. suitable stabilizers, preservatives or buffering salts.

The invention provides a method for the immunisation of warm blooded animals, such as dogs, minks, ferrets and the like which comprises inoculation of the animal with a polyvalent vaccine according to the invention.

The present invention is illustrated by the following Examples.

EXAMPLE 1

BHK-21 cells are grown in monolayers for three to four days until confluency by means of BHK-21 medium containing 10% tryptose phosphate broth and 10% inactivated newborn calf serum in the presence of antibiotics (100 IU of penicillin and 100 micrograms of streptomycin). The confluent monolayer is then infected with the rabies virus strain No. 675 in a multiplicity of infection (m.o.i.) between 0.01 and 1 per cell, and incubated for 45 minutes at 36°C. The virus-containing liquid is then removed, maintenance medium [consisting of BHK-21 medium as described above and 0.2% bovine serum albumin fraction V with antibiotics (i.e. kanamycin 5% in an amount of 2 ml/1000 ml of medium)] added, and the whole medium adjusted to a pH of 7.8 to 8. After 24 hours of virus multiplication the maintenance medium is removed and kept sterile for adding after the infection of the BHK-21 cells with the Onderstepoort avianized distemper virus strain. The infection of the cells by the second virus is performed in the presence of 100 micrograms of diethylaminoethyldextran per ml of medium which is necessary for the adherence of the virus to the cells. Without the use of this polycation, canine distemper virus does not adhere to BHK-21 cells. The multiplicity of infection employed for the second virus is 0.1 per cell. After incubation for one hour at 36°C the virus-containing fluid is removed and the original maintenance medium, which was collected earlier, is added to the cells.

Approximately two to three days later the cell sheet has been destroyed by the action of both viruses and the medium is harvested with the cells and frozen. After thawing, which releases additional amounts of virus from the cells, the cell debris is removed either by means of centrifugation, or by filtration through sterile filters (4 micron pore size). The cell-free virus-containing liquid is frozen and kept at -70°C. A small sample of the cell-free liquid is removed for titration of the viability of the two viruses.

The rabies virus in the harvest is titrated by means of the plaque titration technique on BHK-13 S cells in agarose suspension. This titration technique has the advantage that plaques of the rabies virus only appear; the canine distemper virus does not produce plaques in this system. The latter virus is titrated on African green monkey kidney cells (VERO) by means of the monolayer technique the cells being covered by agarose after infection.

The titres of the living rabies virus vaccine in several harvests varied between $10^{7.5}$ to $10^{8}$ p.m.u./ml and in the same harvests the canine distemper virus vaccine titred between $10^{4.5}$ to $10^{5}$ p.f.u. per ml. These titres for living viruses for vaccine purposes are sufficiently high to guarantee an adequate immune response for preventive vaccination against both viral diseases.

EXAMPLE 2

BHK-21 13 S cells are grown in monolayers for three to four days until confluency by means of BHK-21 medium containing 10% inactivated newborn calf serum in the presence of antibiotics (100 IU of penicillin and 50 micrograms of streptomycin). The confluent monolayer is then infected with the rabies virus strain no. 675 in a multiplicity of infection (m.o.i.) between 0.01 and 1 per cell, and incubated for 45 minutes at 36°C. The virus-containing liquid is then removed, maintenance medium [consisting of BHK-21 medium as described above and 0.2% bovine serum albumin fraction V with antibiotics (i.e. kanamycin in the amount of 50 micrograms/ml of medium)] added, and the whole medium adjusted to a pH of 7.8 to 8. After 24 hours of virus multiplication the maintenance medium is removed. The cells are then infected with the Onderstepoort avianized distemper virus strain in the presence of 100 micrograms of diethylaminoethyldextran per ml of medium for the adherence of the virus to the cells. The m.o.i. employed for the distemper virus is 1 per cell in order to infect theoretically all cells. After incubation for one hour at 36°C the virus-containing fluid is removed and the original maintenance medium, which was collected earlier, is added to the cells.

Approximately 6 hours after the distemper virus infection, the cell sheets are trypsinized and the infected cells are put into spinner culture in the amount of $10^6$ cells/ml of medium. After two to three days of keeping the spinner culture at 33°C, the medium is harvested with the (dead) cells and frozen. After thawing, which releases additional amounts of virus from the cells, the cell debris is removed either by means of centrifugation at 4°C, or by filtration through sterile filters (4 micron pore size) keeping the harvest cool during this procedure in order to avoid a drop in titre of the distemper virus. The cell-free virus-containing liquid is frozen and kept at -70°C. A small sample of the cell-free liquid is removed from titration to determine the viability of the two viruses.

The titration is carried out $\underline{in}$ $\underline{vitro}$ as described in Example 1. The average titres of the rabies virus vaccine varied between $10^{8.5}$ to $10^{9.2}$ p.f.u./ml and the same harvests contained an amount of canine distemper virus vaccine between $10^6$ and $10^7$ p.f.u./ml.

Only living rabies vaccine can be used in this production procedure as the final product containing also distemper virus vaccine loses its potency after inactivation. By proceeding as described above using HEP Flury vaccine instead of the vaccine strain 675 repeatedly similar results have been obtained, but with slightly lower titres of the rabies virus $(10^{8.2} - 10^9$ p.f.u./ml).

0023731

C L A I M S

1. A polyvalent virus vaccine comprising a rabies virus and a canine distemper virus, and substantially free from material emanating from more than one type of tissue cell used to propagate the viruses.

2. A vaccine according to claim 1, characterized in that the rabies virus is the Flury strain or strain no. 675 deposited with the Czechoslovak National Collection of Type Cultures of the Institute of Hygiene and Epidemiology in Prague under CNCTC no. A 04/77.

3. A vaccine according to claim 1 or 2 characterized in that the canine distemper virus is the Wisconsin (FxNO) strain or the Onderstepoort avianized vaccine strain.

4. A vaccine according to claim 1, 2 or 3, characterized in that the rabies virus is strain no. 675 and the canine distemper virus is the Onderstepoort avianized vaccine strain.

5. A vaccine according to any one of the preceding claims characterized in that the titre of rabies virus is $10^{7.5}$ to $10^{9.2}$ p.f.u./ml.

6. A vaccine according to any one of the preceding claims characterized in that the titre of canine distemper virus is $10^{4.5}$ to $10^{7}$ p.f.u./ml.

7. Process for the preparation of a polyvalent virus vaccine comprising at least a living rabies virus and a living canine distemper virus, characterized in that a vaccine is prepared, substantially free from tissue material derived from more than one type of cell used to propagate the viruses, by propagation of the viruses in a single cell system and preparing from the propagation product the polyvalent vaccine.

8. Process according to claim 7, characterized in that it comprises first infecting a suitable single cell system with a rabies virus, propagating the rabies virus and then infecting the cell system with at least a canine distemper virus, propagating the viruses and preparing from the propagation product the polyvalent vaccine.

9. Process according to claims 7-8, characterized in that the single cell system is infected with the rabies virus strain no. 675, deposited with the Czechoslovak National Collection of Type Cultures of the Institute of Hygiene and Epidemiology in Prague under CNCTC no. A 04/77, the ERA strain or the Flury strain.

10. Process according to claims 7-8, characterized in that the single cell system is infected with the canine distemper virus Wisconsin (FxNO) strain or the Onderstepoort avianized vaccine strain.

11. Process according to claims 7-9, characterized in that the single cell system is infected with the rabies virus strain no. 675.

0023731

12. Process according to the claims 7, 8 and 10, characterized in that the canine distemper virus is the Onderstepoort avianized vaccine strain.

13. Process according to any one of the claims 7-12, characterized in that the titre of rabies virus is $10^{7.5}$ to $10^{9.2}$ p.f.u./ml.

14. Process according to any one of the claims 7-12, characterized in that the titre of canine distemper virus is $10^{4.5}$ to $10^7$ p.f.u./ml.

15. Process according to any one of the claims 7-14, characterized in that the viruses are propagated in monolayers of primary or secondary chicken embryo fibroblasts derived from SPF 10 days old chicken embryos or in monolayers derived from other cells of avian origin or in mammalian cells, e.g. in BHK-21 13S suspended cultures.

16. Process according to any one of the claims 7-15, characterized in that the viruses are propagated in BHK-21 monolayers or in BHK-21 13S suspended cultures.

17. Process according to any one of the claims 7-16, characterized in that BHK-21 culture cells are grown in monolayers for three to four days until confluency by means of BHK-21 medium containing 10% tryptose phosphate broth and 10% inactivated newborn calf serum in the presence of antibiotics and the confluent monolayer is then infected with a rabies virus.

18. Process according to any one of the claims 7-17, characterized in that the infection with the rabies virus is effected in a multiplicity of infection between 0.01 and 1 per cell.

0023731

19. Process according to any one of the claims 7-18, characterized in that the cells are infected with the rabies virus by incubation with virus containing liquid for 45-60 minutes at 36°C.

20. Process according to any one of the claims 7-19, characterized in that after infection of the cells with rabies virus, the virus containing liquid is removed, fresh maintenance medium is added, the pH is adjusted to from 7.5 to 8.3 and virus multiplication is allowed to proceed for 12 to 36 hours before infection with canine distemper virus.

21. Process according to any one of the claims 7-20, characterized in that the cells are BHK-21 cells and the infection with the canine distemper virus is performed in the presence of a suitable polycation to ensure adherence of the virus to the cells.

22. Process according to claim 15, characterized in that the polycation is diethylaminoethyldextran.

23. Process according to any one of the claims 7-22, characterized in that the infection of the cells with canine distemper virus is carried out in a multiplicity of infection of 1-0.05 per cell.

24. Process according to any one of the claims 7-23, characterized in that the cells are infected with the canine distemper virus by incubation with virus containing liquid for 1 to 2 hours at 36°C before removal of the virus containing liquid and addition of original maintenance medium.

25. A method for the immunization of warm blooded animals which comprises inoculating the animals with a vaccine as prepared according to any one of the claims 1-6.

Claims for Austria

1. Process for the preparation of a polyvalent virus vaccine comprising at least a living rabies virus and a living canine distemper virus, characterized in that a vaccine is prepared, substantially free from tissue material derived from more than one type of cell used to propagate the viruses, by propagation of the viruses in a single cell system and preparing from the propagation product the polyvalent vaccine.

2. Process according to claim 1, characterized in that it comprises first infecting a suitable single cell system with a rabies virus, propagating the rabies virus and then infecting the cell system with at least a canine distemper virus, propagating the viruses and preparing from the propagation product the polyvalent vaccine.

3. Process according to claims 1-2, characterized in that the single cell system is infected with the rabies virus strain no. 675, deposited with the Czechoslovak National Collection of Type Cultures of the Institute of Hygiene and Epidemiology in Prague under CNCTC no. A 04/77, the ERA strain or the Flury strain.

4. Process according to claims 1-2, characterized in that the single cell system is infected with the canine distemper virus Wisconsin (FxNO) strain or the Onderstepoort avianized vaccine strain.

5. Process according to claims 1-3, characterized in that the single cell system is infected with the rabies virus strain no. 675.

6. Process according to the claims 1, 2 and 4, characterized in that the canine distemper virus is the Onderstepoort avianized vaccine strain.

7. Process according to any one of the claims 1-6, characterized in that the titre of rabies virus is $10^{7.5}$ to $10^{9.2}$ p.f.u./ml.

8. Process according to any one of the claims 1-6, characterized in that the titre of canine distemper virus is $10^{4.5}$ to $10^{7}$ p.f.u./ml.

9. Process according to any one of the claims 1-8, characterized in that the viruses are propagated in monolayers of primary or secondary chicken embryo fibroblasts derived from SPF 10 days old chicken embryos or in monolayers derived from other cells of avian origin or in mammalian cells, e.g. in BHK-21 13S suspended cultures.

10. Process according to any one of the claims 1-9, characterized in that the viruses are propagated in BHK-21 monolayers or in BHK-21 13S suspended cultures.

11. Process according to any one of the claims 1-10, characterized in that BHK-21 culture cells are grown in monolayers for three to four days until confluency by means of BHK-21 medium containing 10% tryptose phosphate broth and 10% inactivated newborn calf serum in the presence of antibiotics and the confluent monolayer is then infected with a rabies virus.

12. Process according to any one of the claims 1-11, characterized in that the infection with the rabies virus is effected in a multiplicity of infection between 0.01 and 1 per cell.

13. Process according to any one of the claims 1-12, characterized in that the cells are infected with the rabies virus by incubation with virus containing liquid for 45-60 minutes at 36$^\circ$C.

14. Process according to any one of the claims 1-13, characterized in that after infection of the cells with rabies virus, the virus containing liquid is removed, fresh maintenance medium is added, the pH is adjusted to from 7.5 to 8.3 and virus multiplication is allowed to proceed for 12 to 36 hours before infection with canine distemper virus.

15. Process according to any one of the claims 1-14, characterized in that the cells are BHK-21 cells and the infection with the canine distemper virus is performed in the presence of a suitable polycation to ensure adherence of the virus to the cells.

16. Process according to claim 15, characterized in that the polycation is diethylaminoethyldextran.

17. Process according to any one of the claims 1-16, characterized in that the infection of the cells with canine distemper virus is carried out in a multiplicity of infection of 1-0.05 per cell.

18. Process according to any one of the claims 1-17, characterized in that the cells are infected with the canine distemper virus by incubation with virus containing liquid for 1 to 2 hours at 36$^\circ$C before removal of the virus containing liquid and addition of original maintenance medium.

19. A method for the immunization of warm blooded animals which comprises inoculating the animals with a
vaccine as prepared according to any one of the claims 1-18.